# EUROPÄISCHE PATENTSCHRIFT

(11) **EP 3 266 375 B1**
(45) Veröffentlichungstag und Bekanntmachung des Hinweises auf die Patenterteilung: **24.07.2019**
(21) Anmeldenummer: 17174052.5
(22) Anmeldetag: 01.06.2017
(51) Int. Cl.: A61B 6/00

(54) **BEWEGUNGSSTEUERUNG FÜR MOBILE RÖNTGENEINRICHTUNG**
MOTION CONTROL FOR MOBILE X-RAY DEVICE
COMMANDE DE MOUVEMENT POUR APPAREIL RADIOGRAPHIQUE PORTABLE

(30) Priorität: 08.07.2016 DE 102016212467
(43) Veröffentlichungstag der Anmeldung: 10.01.2018
(73) Patentinhaber: Siemens Healthcare GmbH, 91052 Erlangen (DE)
(72) Erfinder: Gemmel, Alexander, 91056 Erlangen (DE); Kleinszig, Gerhard, 91301 Forchheim (DE); Wei, Wei, 91301 Forchheim (DE); Weiten, Markus, 90491 Nürnberg (DE)

(56) Entgegenhaltungen:
- DE-A1- 10 153 787
- DE-A1-102014 219 436

## Beschreibung

Die Erfindung betrifft eine mobile Röntgeneinrichtung mit einem auf Rädern verfahrbaren Gerätewagen, der eine Hubvorrichtung aufweist, an der eine Halterung angeordnet ist, an welcher Halterung ein vorzugsweise mehrachsig verstellbarer, insbesondere um einen Drehpunkt drehbarer C-Bogen längs seines Umfangs verschieblich gelagert ist, wobei der C-Bogen an seinem einen Ende eine Röntgenstrahlenquelle und einen dieser gegenüberliegend angeordneten Röntgenstrahlenempfänger aufweist. Mit derartigen Röntgeneinrichtungen werden typischerweise aus einer Serie von unter verschiedenen Projektionswinkeln aufgenommenen 2D-Projektionen eines Körperteils eines Patienten 3D-Bilder des Körperteils rekonstruiert.

Darüber hinaus betrifft die Erfindung ein Verfahren zur Bewegung einer mobilen Röntgeneinrichtung sowie ein Computerprogramm.

Beim Einsatz von mobilen C-Bogen-Röntgeneinrichtungen müssen Röntgenstrahlenquelle und Röntgenstrahlenempfänger so eingestellt werden, dass die Körperregion, die betrachtet werden soll, im Röntgenbild möglichst groß dargestellt wird. In anderen Fällen sollen von einem Untersuchungsobjekt Bilder mit verschiedenen Vergrößerungen aufgenommen werden. Die Objektgröße verändert sich dabei mit dem Abstand zwischen Objekt und Röntgenstrahlenempfänger.

Zur Einstellung des Bildbereiches wird der Gerätewagen zunächst an den Patiententisch herangefahren und der C-Bogen wird so zu dem Patiententisch platziert, dass einen möglichst kleiner Abstand zwischen Objekt und Röntgenstrahlenempfänger besteht. Dies erfolgt, um einen möglichst großen Überblick über die Gesamtanatomie zu erhalten, das Objekt von Interesse zu finden und in der Bildmitte darzustellen. Im Anschluss daran wird erforderlichenfalls das Objekt im Bild bzw. der Bildausschnitt vergrößert bzw. verkleinert. Dies erfolgt durch ein Betätigen der Hubvorrichtung, zumeist in Gestalt einer oftmals bereits motorisierten Hubsäule. Dabei wird der Bildausschnitt durch das Anheben des C-Bogens vergrößert und durch das Absenken des C-Bogens verkleinert. Auf diese Weise wird eine Art Zoomfunktion realisiert, die nachfolgend, in Abgrenzung zu der elektronischen Zoomfunktion innerhalb des aufgenommenen Bildes, als "mechanischer" Zoom bezeichnet wird.

Anders als bei stationären Röntgeneinrichtungen, beispielsweise bodenmontierten oder deckenmontierten Röntgeneinrichtungen, bei denen der Abstand zwischen der Röntgenstrahlenquelle und dem Röntgenstrahlenempfänger veränderbar ist, ergibt sich bei mobilen C-Bogen-Röntgeneinrichtungen, bei denen sich der Abstand zwischen der Röntgenstrahlenquelle und dem Röntgenstrahlenempfänger in der Regel nicht verändern lässt, das Problem, dass nur bei einer strengen AP (anterior posterior)-Stellung des C-Bogens, also einer genau vertikalen Ausrichtung des C-Bogens, bei der Angulations- und Orbitalwinkel jeweils Null sind, das Objekt beim Heben bzw. Absenken der Hubsäule in der Bildmitte bleibt, da die Bewegung dann entlang der Zentralachse erfolgt. Weicht die Stellung (Pose) des C-Bogens davon ab, ist der C-Bogen also schräg gestellt (Orbital-/ Angulationswinkel ungleich Null), ändert sich mit dem Anheben bzw. Absenken der Hubsäule nicht nur, wie gewünscht, der Zoom, also die Darstellungsgröße, sondern auch die Position des Objekts im Bild. Die Zentralachse bewegt sich aus dem Objekt heraus. Das Objekt wandert im Bild bzw. sogar aus dem Bild heraus.

Bisher wurde dieses Problem dadurch gelöst, dass beim Vergrößern mit Hilfe des mechanischen Zooms die C-Bogen-Position manuell nachkorrigiert wird, um das Objekt erneut im Bild zu zentrieren.

Aus DE 10 2014 219 436 A1 und DE 101 53 787 A1 sind mobile C-Bogen-Röntgengeräte bekannt, an deren Gerätewagen motorisch antreibbare Räder bzw. Rollen vorgesehen sind. Diese werden angetrieben, um die mit Hilfe der Halterung erfolgende Positionierung des C-Bogens zu unterstützen.

Eine Aufgabe der Erfindung ist es, die Handhabung des mechanischen Zooms bei mobilen Röntgeneinrichtungen zu vereinfachen. Diese Aufgabe wird durch eine mobile Röntgeneinrichtung nach Anspruch 1 bzw. durch ein Verfahren nach Anspruch 2 bzw. durch ein Computerprogramm nach Anspruch 6 gelöst. Vorteilhafte Ausführungen der Erfindung sind in den Unteransprüchen angegeben.

Die im Folgenden im Zusammenhang mit der mobilen Röntgeneinrichtung erläuterten Vorteile und Ausgestaltungen gelten sinngemäß auch für das erfindungsgemäße Verfahren bzw. das Computerprogramm und umgekehrt.

Erfindungsgemäß ist eine Bewegungssteuerung vorgesehen, mit der in jeder gegebenen Ausgangspose des C-Bogens eine Bewegung des C-Bogens so gesteuert wird, dass die zwischen Röntgenstrahlenquelle und Röntgenstrahlenempfänger verlaufende Zentralachse raumfest ist und bleibt.

Unter einer Pose wird allgemein die Position und Orientierung eines Objekts im Raum verstanden. Die hier genannte (relative) Pose des C-Bogens bedeutet die relative Position und Orientierung, genauer gesagt die Lage des C-Bogens bezüglich der C-Bogen-Halterung, die mit Hilfe der Hubvorrichtung angehoben und abgesenkt werden kann. Nachfolgende werden die Begriffe Stellung und Pose teilweise synonym verwendet. Gleiches gilt für die Begriffe Hubvorrichtung und Hubsäule, wobei die vertikale Hubbewegung des C-Bogen-Halters und damit des C-Bogens selbst auch mit Hilfe anderer Mittel als eine Hubsäule verwirklicht werden kann, die Hubsäule hier also als lediglich beispielhaft anzusehen ist.

Unter der Zentralachse wird die Strahlmittelachse verstanden, also die Mittelachse des von der Röntgenstrahlenquelle ausgesendeten Röntgenstrahlbündels.

Eine Grundidee der vorliegenden Erfindung ist es, das Objekt, von dem die Röntgenaufnahmen gemacht werden sollen, immer im Zentralstrahl zu halten. Dies wird dadurch verwirklicht, dass der C-Bogen als Träger von Röntgenstrahlenquelle und Röntgen strahlenempfänger auf hierfür geeignete Weise bewegt wird. Bei dieser Bewegung handelt es sich insbesondere um eine Bewegung des Gerätwagens (und damit der Halterung und des C-Bogens) in horizontaler Richtung und/oder um eine Bewegung des C-Bogens in vertikaler Richtung, bewirkt durch das Anheben bzw. Absenken der Halterung mittels der Hubsäule. Zu diesem Zweck ist es vorgesehen, dass die entsprechenden Bewegungsmittel über motorisierte Antriebe verfügen und eine geeignete Ansteuerung dieser Antriebe mittels einer Bewegungssteuerung erfolgt. Da die Bewegung motorisch erfolgt, ist eine automatisierbare Kompensations- oder Ausgleichsbewegung möglich, insbesondere in Form einer Nachführung bzw. einer Stellungskorrektur des C-Bogens. Dies entspricht auch der bevorzugten Ausführungsform der Erfindung.

Im Grunde wäre es ebenfalls möglich, das Objekt dauerhaft im Zentralstrahl zu halten, indem das Objekt auf hierfür geeignete Weise bewegt wird, insbesondere durch eine Bewegung des Patiententisches in horizontaler und/oder vertikaler Richtung. Bei der hier beschriebenen Konstellation, insbesondere der Verwendung einer ohnehin mobilen Röntgeneinrichtung mit einem motorisch angetriebenen Hubsäule und motorisch antreibbaren Rädern, ist die als vorteilhaft beschriebene Lösung ohne eine Bewegung des Objektes einfacher und mit weniger Aufwand realisierbar, als das deutlich aufwendigere Nachführen des in der Regel sehr schweren Patiententisches.

Die erfindungsgemäße Steuerung der Bewegung des C-Bogens derart, dass die Zentralachse ihre Position im Raum beibehält, erfolgt ausschließlich während der Ausführung des eingangs geschilderten mechanischen Zooms, d.h. im Fall einer Bewegung des Röntgenstrahlenempfängers auf das Objekt zu oder von dem Objekt weg, also während der Röntgenstrahlenempfänger auf das Objekt zu (Zoom out) oder vom Objekt weg (Zoom in) bewegt wird. Während anderer Zeiträume findet die Ausgleichsbewegung vorzugsweise nicht statt. Insbesondere findet dann kein horizontales Verfahren des Gerätewagens statt, insbesondere aus Sicherheitsgründen.

Dabei handelt es sich bei der gesteuerten Bewegung des C-Bogens um eine vertikale und/oder horizontale Verfahrbewegung des C-Bogens. Vorzugsweise erfolgt ausschließlich eine vertikale und/oder horizontale Verfahrbewegung des C-Bogens, d.h. keine andere Ausgleichsbewegung. Im Besonderen werden dann nur die Hubsäule und/oder die Antriebsräder angesteuert, während die Stellung des C-Bogens zur Halterung unverändert bleibt.

Es wird also eine mobile Röntgeneinrichtung bereitgestellt, bei der in jeder gegebenen Pose des C-Bogens im Fall einer Bewegung des Röntgenstrahlenempfängers auf das Objekt zu oder von dem Objekt weg eine vertikale und/oder horizontale Verfahrbewegung des C-Bogens erfolgt derart, dass die Zentralachse raumfest ist bzw. ihre Position im Raum beibehält.

Mit Hilfe der Erfindung kann eine einfache automatische Korrektur der Position des Objektes im Bild erfolgen. Eine aufwendige Ansteuerung diverser Achsen des C-Bogens ist dafür nicht notwendig. Bei jedem mechanischen Zoom, insbesondere durch Anheben bzw. Absenken der Hubsäule, erfolgt ein entsprechendes horizontales Verfahren des C-Bogens, so dass das Objekt immer im Zentralstrahl gehalten wird und daher stets zentriert im Bild erscheint. Diese Ausgleichsbewegung des C-Bogens kann synchron, im Sinne von im wesentlichen gleichzeitig, oder asynchron, d. h. mit einer nicht zu vernachlässigenden Zeitdifferenz, erfolgen. Auch eine asynchrone Ausgleichsbewegung ist akzeptabel, sofern der Zeitpunkt der tatsächlich Röntgenaufnahme hierauf abgestimmt ist, die Röntgenaufnahme also erst dann erfolgt, wenn die Ausgleichsbewegung abgeschlossen ist.

Mit Hilfe einer Röntgeneinrichtung mit motorisierten Rädern ist es möglich, die C-Bogen-Position, insbesondere die Lage des C-Bogens im Raum bei gleichbleibender Orientierung, automatisch anzupassen. Durch eine geeignete Bewegung des C-Bogens kann das Objekt immer im Zentralstrahl gehalten werden. Hubsäulen- und/oder Radposition werden erfindungsgemäß so geändert, dass der Zentralstrahl eine feste Position im Raum hat. Anders ausgedrückt wird mit Hilfe einer Motorisierung der Hubsäule und der angetriebene Räder der C-Bogen so bewegt, dass die mechanische Zoom-Bewegung immer entlang der Zentralachse erfolgt, unabhängig von der Pose des C-Bogens. Bei den Rädern kann es sich um eine Anzahl omnidirektional antreibbare Räder oder eine Anzahl anderer Räder mit geeigneten Antrieben handeln. Wesentlich ist, dass sich der Gerätewagen und damit der darauf befestigte C-Bogen in beliebiger Fahrtrichtung bewegen lässt, also beispielsweise vor und zurück als auch seitlich. Eine freie Verfahrbarkeit in alle Richtungen ist für die gewünschte Ausgleichs- oder Korrekturbewegung des C-Bogens in solchen Fällen von Bedeutung, wenn Angulations- und Orbitalwinkel der C-Bogen-Pose ungleich Null sind. Bei den motorischen Antrieben der Hubsäule bzw. der Räder handelt es sich vorzugsweise um präzise ansteuerbare Stellmotoren bzw. Schrittmotoren.

In Abhängigkeit von der Ausgangspose muss der C-Bogen auf unterschiedliche Weise angesteuert und bewegt werden. In einem ersten Spezialfall einer strengen AP-Pose (vertikaler C-Bogen) muss lediglich die Hubsäule angesteuert werden. In einem anderen Spezialfall einer streng lateralen Pose (horizontaler C-Bogen) müssen nur die Räder angetrieben werden.

Die Erfindung geht von einer Situation aus, in der eine bestimmte Ausgangspose des C-Bogens vorgegeben ist. Dabei kann es sich um die AP-Pose oder aber um eine beliebige andere ("schräge") Pose handeln. Die (gesteuerte) Bewegung des C-Bogens, welche auf die in der Erfindung beschriebene besondere Art und Weise erfolgt, bezieht sich also jeweils auf eine solche Pose des C-Bogens und erfolgt während des (vorzugsweise unveränderten) Vorliegens einer solchen C-Bogen-Stellung. Mit anderen Worten wird davon ausgegangen, dass der C-Bogen eine bestimmte Orientierung eingenommen hat, nämlich in Bildgebungsrichtung ausgerichtet ist, also die Stellung der Drehwinkel (angular-orbital) feststeht und auch sonst keinerlei Schwenk-, Dreh- oder Kippbewegungen des C-Bogens selbst mehr erfolgen, es sich bei der gesteuerten Bewegung des C-Bogens also ausschließlich um eine vertikale und/oder horizontale Verfahrbewegung des C-Bogens handelt.

Eine solche weitere Bewegung des C-Bogens wird dann erfindungsgemäß so gesteuert, dass die Zentralachse raumfest ist. Insbesondere ist dies interessant für den Fall einer Bewegung des Röntgenstrahlenempfängers auf das Objekt zu bzw. von dem Objekt weg, wie es bei einem mechanischen Zoom der Fall ist. Eine solche Bewegung erfolgt in der Regel durch eine Höhenverstellung mittels der Hubsäule, also eine vertikale Verfahrbewegung des C-Bogens. Alternativ hierzu ist es aber ebenfalls möglich, dass eine solche Bewegung des Röntgenstrahlenempfängers auf das Objekt zu bzw. von dem Objekt weg durch das Bewegen des Objekts selbst, z. B. eine Anheben oder Absenken des Patiententisches verwirklicht wird. Auch in diesem Fall bleibt es bei der raumfesten Zentralachse. Die vertikale und/oder horizontale Verfahrbewegung des C-Bogens wird erfindungsgemäß so gesteuert, dass die Zentralachse ihre Raumposition beibehält. Im Ergebnis bedeutet dies, dass die Bewegung auf das Objekt bzw. von dem Objekt weg stets entlang der (raumfesten) Zentralachse erfolgt.

Vorzugsweise handelt es sich bei der vertikalen und/oder horizontalen Verfahrbewegung des C-Bogens jeweils um eine indirekte Verfahrbewegung. Das bedeutet, dass der C-Bogen selbst nicht in seiner Halterung verfahren wird, er also seine Pose beibehält. Der C-Bogen wird dennoch relativ zu dem Objekt bewegt, indem der Gerätewagen mitsamt Halterung und C-Bogen und/oder die Halterung mit dem C-Bogen bewegt über die Hubsäule bewegt wird.

In einer bevorzugten Ausführungsform der Erfindung wird eine solche Bewegungssteuerung durch eine entsprechende Antriebssteuerung zur Steuerung des motorischen Antriebs der Hubsäule sowie eine entsprechende Antriebssteuerung zur Steuerung des motorischen Antriebs der Räder verwirklicht, indem der motorische Antrieb der Hubsäule bzw. der motorische Antrieb der Räder automatisch unter Beibehaltung der raumfesten Zentralachse angesteuert wird. Insbesondere die zuletzt genannte Möglichkeit ist bei der Anwendung des mechanischen Zooms von Interesse. Der durch ein Anheben bzw. Absenken des C-Bogens mittels der Hubsäule bewirkte mechanische Zoom wird dadurch so ausgeführt, dass das Objekt stets in der Zentralachse gehalten wird, also nicht aus dem Bild herauswandert, da aufgrund der entsprechenden synchronen Korrekturbewegung, nämlich der horizontalen Verfahrbewegung des C-Bogens mittels der angetriebenen Räder, die Bewegung des Röntgendetektors auf das Objekt zu bzw. von dem Objekt weg auf der Zentralachse erfolgt. In diesem Fall ist also die Bewegungsrichtung gleich der Richtung des Zentralstrahls. Das entspricht der bevorzugten Ausführungsform der Erfindung.

Die Erfindung schlägt eine Stellungskorrektur vor bzw. ein Nachführen des C-Bogens aufgrund und während des mechanischen Zooms, also dem Bewegen des Röntgenstrahlenempfängers auf das Objekt zu bzw. von dem Objekt weg. Mit anderen Worten wird der C-Bogen entsprechend der Zoom-Bewegung, vorzugsweise synchron zu der Zoom-Bewegung, so nachgeführt, dass der interessierende Objektbereich (ROI) zu jedem Zeitpunkt innerhalb eines Strahlkegels des Röntgenstrahlbündels liegt. Die Verschiebung der Zentralachse relativ zu dem Untersuchungsobjekt wird durch die Ausgleichsbewegung des C-Bogens (oder des Objekt- bzw. Patiententisches) ausgeglichen bzw. kompensiert, so dass vorzugsweise immer die gleichen Teilbereiche des Untersuchungsobjekts von der Röntgenstrahlung durchstrahlt werden bzw. der Zentralstrahl auch bei verschiedenen Stellungen des mechanischen Zooms stets durch das Untersuchungszentrum verläuft.

Die zum Zweck dieser Stellungskorrektur des C-Bogens zurückzulegenden Verfahrwege der Hubsäule bzw. des Gerätewagens in vertikaler bzw. horizontaler Richtung werden von der Bewegungssteuerung in Abhängigkeit von den vertikalen bzw. horizontalen Verfahrbewegungen vorgegeben, die der C-Bogen aufgrund und während des mechanischen Zooms vornimmt. Der Bewegungssteuerung werden hierzu die notwendigen Informationen zur Verfügung gestellt. Bei diesen Informationen kann es sich beispielsweise um Positionsdaten des Gerätewagens oder der Halterung oder um Steuerinformationen handeln. In einer bevorzugten Ausführungsform der Erfindung wird also zunächst die tatsächliche Zoom-Bewegung des Röntgenstrahlenempfängers ermittelt, beispielsweise in Gestalt einer vertikalen Anhebe- oder Absenkbewegung. Anschließend bzw. zeitlich synchron erfolgt das Ausgleichen durch die entsprechende Gegen- oder Ausgleichsbewegung, angesteuert unter Verwendung der Daten der Zoom-Bewegung.

Mit der Erfindung wird die Handhabung des mechanischen Zooms bei mobilen Röntgeneinrichtungen deutlich vereinfacht. Im Besonderen lässt sich eine - zumindest in Bezug auf den mechanischen Zoom - vollautomatisch arbeitende Röntgeneinrichtung bereitstellen, die nicht, wie üblich, ortsfest, also am Aufstellungsort starr an Decke, Wand oder Fußboden montiert, sondern mobil ausgeführt ist, insbesondere auf dem Fußboden in beliebige Richtungen verfahrbar ist.

Insoweit die Erfindung, im Besonderen die Bewegungssteuerung, in oder mit einer Datenverarbeitungseinheit realisiert ist, weist diese Datenverarbeitungseinheit vorzugsweise eine Anzahl von Funktionsmodulen auf, wobei jedes Funktionsmodul ausgebildet ist zur Durchführung einer bestimmten Funktion oder einer Anzahl bestimmter Funktionen gemäß dem beschriebenen Verfahren. Bei den Funktionsmodulen kann es sich um Hardwaremodule oder Softwaremodule handeln. Mit anderen Worten kann die Erfindung, soweit es die Datenverarbeitungseinheit betrifft, entweder in Form von Computerhardware oder in Form von Computersoftware oder in einer Kombination aus Hardware und Software verwirklicht werden. Soweit die Erfindung in Form von Software, also als Computerprogramm, verwirklicht ist, werden sämtliche beschriebenen Funktionen durch Computerprogrammanweisungen realisiert, wenn das Computerprogramm auf einem Rechner mit einem Prozessor ausgeführt wird. Die Computerprogrammanweisungen sind dabei auf an sich bekannte Art und Weise in einer beliebigen Programmiersprache verwirklicht und können dem Rechner in beliebiger Form bereitgestellt werden, beispielsweise in Form von Datenpaketen, die über ein Rechnernetz übertragen werden, oder in Form eines auf einer Diskette, einer CD-ROM oder einem anderen Datenträger gespeicherten Computerprogramms.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Dabei zeigen:
FIG 1 einen mechanischen Zoom bei einer Röntgeneinrichtung mit C-Bogen nach dem Stand der Technik,
FIG 2 einen mechanischen Zoom bei einer erfindungsgemäßen Röntgeneinrichtung mit C-Bogen.

Sämtliche Figuren zeigen die Erfindung lediglich schematisch und mit ihren wesentlichen Bestandteilen. Gleiche Bezugszeichen entsprechen dabei Elementen gleicher oder vergleichbarer Funktion.

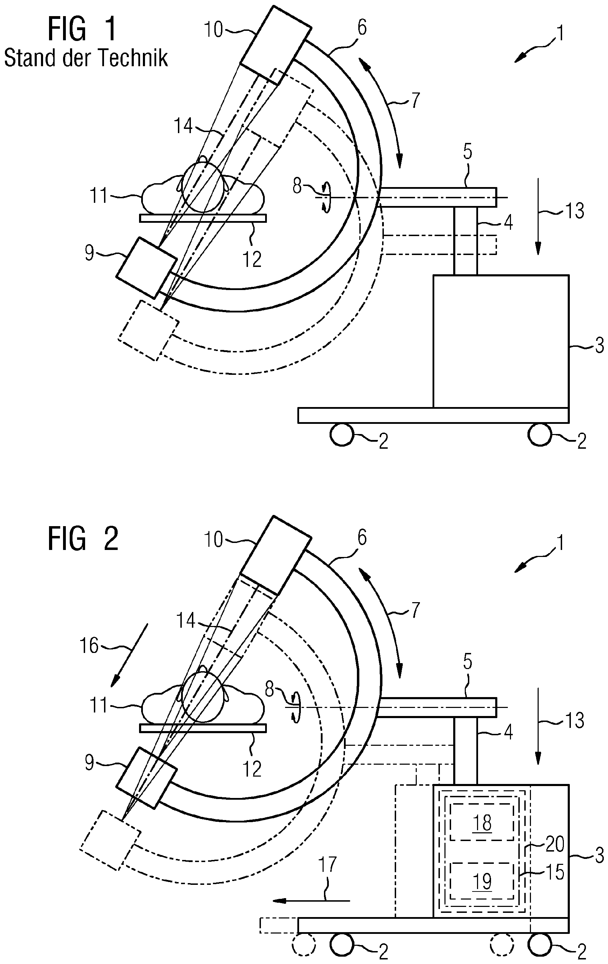

Die FIG 1 und 2 zeigen eine mobile Röntgeneinrichtung 1 mit einem auf Rädern 2 verfahrbaren Gerätewagen 3. Der Gerätewagen 3 weist eine Hubvorrichtung in Form einer Hubsäule 4 auf, an der eine Halterung 5 angeordnet ist. An der Halterung 5 ist ein mehrachsig verstellbarer, um einen Drehpunkt drehbarer C-Bogen 6 (Orbitalwinkel 7) längs seines Umfangs verschieblich gelagert (Angulationswinkel 8). Der C-Bogen 6 weist an seinem einen Ende eine Röntgenstrahlenquelle 9 und einen dieser gegenüberliegend angeordneten Röntgenstrahlenempfänger 10 auf. Der C-Bogen 6 ist nahe eines zu untersuchenden Objektes 11 platziert, wobei es sich bei dem Untersuchungsobjekt 11 beispielsweise um ein Körperteil eines Patienten handelt. Das Objekt 11 ist auf einem Untersuchungstisch 12 platziert. Der C-Bogen 6 ist schräg gestellt. Orbitalwinkel 7 und Angulationswinkel 8 sind ungleich Null und definieren somit die Ausgangspose des C-Bogens 6 relativ zu der Halterung 5.

Im vorliegenden Beispielfall soll ein Zoom-out erfolgen. Ausgehend von einem großen Abstand zwischen Objekt 11 und Röntgenstrahlenempfänger 10 wird bei einem mechanischen Zoom, verwirklicht durch ein motorisiertes vertikales Absenken des C-Bogens 6 mit Hilfe der Hubsäule 4, der Abstand zwischen dem Röntgenstrahlenempfänger 10 und dem Objekt 11 verringert. Eine solche Hubbewegung ist mit Pfeil 13 bezeichnet. Die Ausgangsposition mit großem Objektabstand ist mit durchgezogenen Linien, die Endposition mit kleinem Objektabstand nach Abschluß des mechanischen Zooms ist mit durchbrochenen Linien dargestellt. Bei der in FIG 1 dargestellten Lösung, wie sie aus dem Stand der Technik bekannt ist, ändert sich die Position des Objekts 11 im Bild. Die Zentralachse 14 bewegt sich aus dem Objekt 11 heraus.

Bei der in FIG 2 dargestellten Lösung ist das nicht der Fall, da dort die Räder 2 motorisch antreibbar und bezüglich ihrer Fahrtrichtung steuerbar sind und unter Verwendung einer entsprechenden Bewegungssteuerung 15 eine synchrone Ausgleichsbewegung des C-Bogens 6 stattfindet derart, dass die Zentralachse 14 ortsfest ist und bleibt. Das Objekt 11 wird daher in der Zentralachse 14 gehalten. Die Zoom-Bewegung 16 des C-Bogens 6 erfolgt entlang der Zentralachse 14.

Die Ausgleichsbewegung des C-Bogens 6 erfolgt über ein horizontales Verfahren des Gerätewagens 3, symbolisiert in FIG 2 durch Pfeil 17. Die Verfahrbewegung des Gerätewagens 3 erfolgt dabei hinsichtlich des zurückzulegenden Weges in Abhängigkeit von der Länge des vertikalen Hubs während des mechanischen Zooms und hinsichtlich der Richtung, in die der Gerätewagen 3 zu verfahren ist, in Abhängigkeit von der Hubrichtung (Anheben oder Absenken) und der Pose des C-Bogens (Orbitalwinkel 7 und Angulationswinkel 8).

Sowohl die Hubsäule 4 als auch die bezüglich ihrer Fahrtrichtung ausrichtbaren Räder 2 verfügen über elektromotorische Antriebe in Form von Servomotoren, die mit einer Bewegungssteuerung verbunden sind. Die Bewegungssteuerung 15 ist als Computerprogramm realisiert, das in einer Recheneinrichtung 20 ausgeführt wird, wobei diese Recheneinrichtung 20 vorzugsweise ein Teil der zentralen Steuereinheit der Röntgeneinrichtung 1 ist. In der Bewegungssteuerung 15 werden die benötigten Informationen zusammengeführt. Außerdem werden in der Bewegungssteuerung 15 die entsprechenden Steuersignale für die Antriebe erzeugt. Zu diesem Zweck umfasst die Bewegungssteuerung 15 eine erste Antriebssteuerung 18 zur Steuerung des motorischen Antriebs (nicht dargestellt) der Hubsäule 4 und eine mit der ersten Antriebssteuerung 18 zusammenwirkende zweite Antriebssteuerung 19 zur Steuerung des motorischen Antriebs (nicht dargestellt) der Räder 2. Die Ansteuerung erfolgt dabei jeweils unter Beibehaltung der raumfesten Zentralachse 14, wobei die beiden Antriebssteuerungen 18, 19 die hierfür notwendigen Informationen austauschen.

Die hier skizzierte Idee der Erfindung ist es, bei einem mechanischen Zoom, also einem Vergrößern oder Verkleinern des Bildausschnittes durch ein Anheben oder Absenken des C-Bogens 6 mittels der Hubsäule 4, eine Ausgleichsbewegung des C-Bogens 6 vorzusehen derart, dass die Zentralachse 14 raumfest bleibt, so dass sich das Objekt 11 nicht im Bild verschiebt. Hierzu werden motorisch angetriebene Räder 2 benutzt, mit deren Hilfe der Gerätewagen 3 entsprechend der benötigten Ausgleichsbewegung verfahren wird. Allgemein gilt, dass das Objekt 11 immer in der Zentralachse 14 gehalten wird. Dies geschieht durch Bewegung der zugehörigen Achsen des C-Bogens 6 in Abhängigkeit von dessen Ausgangspose.

Obwohl die Erfindung im Detail durch das bevorzugte Ausführungsbeispiel näher illustriert und beschrieben wurde, so ist die Erfindung nicht auf die offenbarten Beispiele eingeschränkt und andere Variationen können vom Fachmann hieraus abgeleitet werden, ohne den Schutzumfang der Erfindung zu verlassen.

### Bezugszeichenliste

- 1: Röntgeneinrichtung
- 2: Rad
- 3: Gerätewagen
- 4: Hubsäule
- 5: Halterung
- 6: C-Bogen
- 7: Orbitalwinkel
- 8: Angulationswinkel
- 9: Röntgenstrahlenquelle
- 10: Röntgenstrahlenempfänger
- 11: Objekt
- 12: Tisch
- 13: Hubbewegung (Vertikalrichtung)
- 14: Zentralachse
- 15: Bewegungssteuerung
- 16: Zoom-Bewegung
- 17: Verfahrbewegung (Horizontalrichtung)
- 18: erste Antriebssteuerung
- 19: zweite Antriebssteuerung
- 20: Recheneinrichtung

## Patentansprüche

1. Mobile Röntgeneinrichtung (1) mit einem auf Rädern (2) verfahrbaren Gerätewagen (3), der eine Hubvorrichtung (4) aufweist, an der eine Halterung (5) angeordnet ist, an welcher Halterung (5) ein C-Bogen (6) längs seines Umfangs verschieblich gelagert ist, wobei der C-Bogen (6) eine Röntgenstrahlenquelle (9) und einen dieser gegenüberliegend angeordneten Röntgenstrahlenempfänger (10) aufweist, wobei eine Bewegungssteuerung (15) vorgesehen ist, die eine erste Antriebssteuerung (18) zur Steuerung eines motorischen Antriebs der Hubvorrichtung (4) zum Erzeugen einer vertikalen Verfahrbewegung (13) des C-Bogens (6) und eine zweite, mit der ersten Anriebssteuerung zusammenwirkende Antriebssteuerung (19) zur Steuerung eines motorischen Antriebs der Räder (2) zum Erzeugen einer horizontalen Verfahrbewegung (17) des C-Bogens (6) umfasst, **dadurch gekennzeichnet, dass** im Fall einer motorischen Bewegung des Röntgenstrahlempfängers (10) auf das Objekt (11) zu oder von dem Objekt (11) weg, in jeder gegebenen Pose des C-Bogens (6) mittels der Bewegungssteuerung eine Ausgleichsbewegung des C-Bogens in Form einer vertikalen Verfahrbewegung (13) und/oder einer horizontale Verfahrbewegung (17) so erfolgt, dass die zwischen Röntgenstrahlenquelle (9) und Röntgenstrahlenempfänger (10) verlaufende Zentralachse (14) raumfest bleibt, indem die erste Antriebssteuerung (18) den motorischen Antrieb der Hubvorrichtung (4) automatisch unter Beibehaltung der raumfesten Zentralachse (14) ansteuert und/oder indem die zweite Antriebssteuerung (19) den motorischen Antrieb der Räder (2) automatisch unter Beibehaltung der raumfesten Zentralachse (14) ansteuert.

2. Verfahren zur Bewegung einer mobilen Röntgeneinrichtung (1) mit einem auf Rädern (2) verfahrbaren Gerätewagen (3), der eine Hubvorrichtung (4) aufweist, an der eine Halterung (5) angeordnet ist, an welcher Halterung (5) ein C-Bogen (6) längs seines Umfangs verschieblich gelagert ist, wobei der C-Bogen (6) eine Röntgenstrahlenquelle (9) und einen dieser gegenüberliegend angeordneten Röntgenstrahlenempfänger (10) aufweist, und mit einer Bewegungssteuerung (15) zur Steuerung einer Bewegung des C-Bogens (6), wobei die Bewegungssteuerung (15) eine erste Antriebssteuerung (18) zur Steuerung eines motorischen Antriebs der Hubvorrichtung (4) zum Erzeugen einer vertikalen Verfahrbewegung (13) des C-Bogens (6) und eine mit der ersten Antriebssteuerung zusammenwirkende zweite Antriebssteuerung (19) zur Steuerung eines motorischen Antriebs der Räder (2) zum Erzeugen einer horizontalen Verfahrbewegung (17) des C-Bogens (6) umfasst, **dadurch gekennzeichnet, dass** in jeder gegebenen Pose des C-Bogens (6) im Fall einer motorischen Bewegung des Röntgenstrahlenempfängers (10) auf das Objekt (11) zu oder von dem Objekt (11) weg eine Ausgleichsbewegung des C-Bogens (6) in Form einer vertikalen Verfahrbewegung (13) und/oder einer horizontalen Verfahrbewegung (17) des C-Bogens (6) so gesteuert wird, dass die zwischen Röntgenstrahlenquelle (9) und Röntgenstrahlenempfänger (10) verlaufende Zentralachse (14) raumfest ist, d.h. ihre Position im Raum beibehält, wobei die erste Antriebssteuerung (18) den motorischen Antrieb der Hubvorrichtung (4) automatisch unter Beibehaltung der raumfesten Zentralachse (14) ansteuert und/oder wobei die zweite Antriebssteuerung (19) den motorischen Antrieb der Räder (2) automatisch unter Beibehaltung der raumfesten Zentralachse (14) ansteuert.

3. Verfahren nach Anspruch 2, wobei die Ausgleichsbewegung des C-Bogens (6) ausschließlich in Form einer vertikalen und/oder horizontalen Verfahrbewegung des C-Bogens (6) erfolgt.

4. Verfahren nach Anspruch 2 oder 3, wobei zur Durchführung der Ausgleichsbewegung des C-Bogens (6) ausschließlich die motorischen Antriebe der Hubvorrichtung (4) und der Räder (2) angesteuert werden.

5. Verfahren nach einem der Ansprüche 2 bis 4, wobei die Ausgleichsbewegung des C-Bogens (6) ausschließlich während einer Bewegung des Röntgenstrahlenempfängers (10) auf das Objekt (11) zu oder von dem Objekt (11) weg erfolgt.

6. Computerprogramm, das ein Verfahren nach einem der Ansprüche 2 bis 5 durchführt, wenn es auf einer Recheneinrichtung (20) ausgeführt wird, die Teil einer zentralen Steuereinheit einer mobilen Röntgeneinrichtung (1) nach Anspruch 1 ist.

## Claims

1. Mobile X-ray device (1) having an equipment cart (3) that is movable on wheels (2) and has a lifting device (4) on which a support assembly (5) is arranged, on which support assembly (5) a C-arm (6) is mounted so as to be displaceable along its circumference, wherein the C-arm (6) has an X-ray source (9) and an X-ray receiver (10) arranged opposite said X-ray source (9), wherein a motion controller (15) is provided which comprises a first drive controller (18) for controlling a motorised drive of the lifting device (4) in order to generate a vertical positioning movement (13) of the C-arm (6) and a second drive controller (19), which interacts with the first drive controller, for controlling a motorised drive of the wheels (2) in order to generate a horizontal positioning movement (17) of the C-arm (6),
**characterised in that** in the case of a motorised movement of the X-ray receiver (10) to and fro with respect to the object (11), in any given pose of the C-arm (6) the motion controller (15) is used to carry out a compensation movement of the C-arm in the form of a vertical positioning movement (13) and/or a horizontal positioning movement (17) such that the central axis running between the X-ray source (9) and X-ray receiver (10) remains space-fixed by the first drive controller (18) actuating the motorised drive of the lifting device (4) automatically while maintaining the space-fixed central axis (14) and/or by the second drive controller (19) actuating the motorised drive of the wheels (2) automatically while maintaining the space-fixed central axis (14).

2. Method for moving a mobile X-ray device (1) having an equipment cart (3) that is movable on wheels (2) and has a lifting device (4) on which a support assembly (5) is arranged, on which support assembly (5) a C-arm (6) is mounted so as to be displaceable along its circumference, wherein the C-arm (6) has an X-ray source (9) and an X-ray receiver (10) arranged opposite said X-ray source (9), and has a motion controller (15) for controlling a movement of the C-arm (6), wherein the motion controller (15) comprises a first drive controller (18) for controlling a motorised drive of the lifting device (4) in order to generate a vertical positioning movement (13) of the C-arm (6) and a second drive controller (19), which interacts with the first drive controller (18), for controlling a motorised drive of the wheels (2) in order to generate a horizontal positioning movement (17) of the C-arm (6),
**characterised in that**
in any given pose of the C-arm (6) in the case of a motorised movement of the X-ray receiver to and fro with respect to the object (11), a compensation movement of the C-arm in the form of a vertical positioning movement (13) and/or a horizontal positioning movement (17) of the C-arm (6) is controlled such that the central axis (14) running between the X-ray source (9) and X-ray receiver (10) remains space-fixed, i.e. maintains its position in the space,
wherein the first drive controller (18) actuates the motorised drive of the lifting device (4) automatically while maintaining the fixed-space central axis (14) and/or wherein the second drive controller (19) actuates the motorised drive of the wheels (2) automatically while maintaining the fixed-space central axis (14).

3. Method according to claim 2, wherein the compensation movement of the C-arm (6) is carried out only in the form of a vertical and/or horizontal positioning movement of the C-arm (6).

4. Method according to claim 2 or 3, wherein only the motorised drives of the lifting device (4) and the wheels (2) are actuated in order to carry out the compensation movement of the C-arm (6).

5. Method according to one of claims 2 to 4, wherein the compensation movement of the C-arm (6) is only carried out during a movement of the X-ray receiver (10) to or fro with respect to the object (11).

6. Computer program which performs a method according to one of claims 2 to 5 when it is executed on a computing device (20), which is part of a central control unit of a mobile X-ray device (1) according to claim 1

## Revendications

1. Dispositif (1) radiographique mobile, comprenant un chariot (3) d'appareil pouvant se déplacer sur des roues (2) et ayant un système (4) de levage, sur lequel est disposée une fixation (5), sur laquelle fixation (5) un arceau en C (6) est monté de manière à se déplacer le long de son pourtour, l'arceau en C (6) ayant une source (9) de rayon X et un récepteur (10) de rayon X disposé en opposition à celle-ci, dans lequel il est prévu une commande (15) de déplacement, qui comprend une première commande (18) d'entraînement pour commander un entraînement motorisé du système (4) de levage, afin de produire un déplacement (13) vertical de l'arceau en C (6), et une deuxième commande (19) d'entraînement coopérant avec la première commande d'entraînement pour commander un entraînement motorisé des roues (2), afin de produire un déplacement (17) horizontal de l'arceau en C (6), **caractérisé en ce que**, dans le cas d'un déplacement motorisé du récepteur (10) de rayon X, en allant vers l'objet (11) ou en s'en éloignant, dans chaque pose donnée de l'arceau en C (6), il s'effectue, au moyen de la commande de déplacement, un déplacement de compensation de l'arceau en C sous la forme d'un déplacement (13) vertical et/ou d'un déplacement (17) horizontal, de manière à ce que l'axe (14) central s'étendant entre la source (9) de rayon X et le récepteur (10) de rayon X reste fixe dans l'espace par le fait que la première commande (18) d'entraînement commande l'entraînement motorisé du système (4) de levage automatiquement, en conservant l'axe (14) central fixe dans l'espace, et/ou par le fait que la deuxième commande (19) d'entraînement commande l'entraînement motorisé des roues (2) automatiquement en conservant l'axe (14) central fixe dans l'espace.

2. Procédé de déplacement d'un dispositif (1) radiographique mobile, comprenant un chariot (3) d'appareil pouvant être déplacé sur des roues (2) et ayant un système (4) de levage, sur lequel est disposée une fixation (5), sur laquelle fixation (5) un arceau en C (6) est monté de manière à pouvoir se déplacer le long de son pourtour, l'arceau en C (6) ayant une source (9) de rayon X et un récepteur (10) de rayon X disposé à l'opposé de celle-ci, et comprenant une commande (15) de déplacement pour commander un déplacement de l'arceau en C (6), la commande (15) de déplacement comprenant une première commande (18) d'entraînement pour commander un entraînement motorisé du système (4) de levage, afin de produire un déplacement (13) vertical de l'arceau en C (6), et une deuxième commande (19) d'entraînement coopérant avec la première commande d'entraînement pour commander un entraînement motorisé des roues (2), afin de produire un déplacement (17) horizontal de l'arceau en C (6), **caractérisé en ce que**, dans chaque pose donnée de l'arceau en C (6), dans le cas d'un déplacement motorisé du récepteur (10) de rayon X, se rapprochant de l'objet (11) ou s'en éloignant, on commande un déplacement de compensation de l'arceau en C (6) sous la forme d'un déplacement (13) vertical et/ou un déplacement (17) horizontal de l'arceau en C (6), de manière à ce que l'axe (14) central s'étendant entre la source (9) de rayon X et le récepteur (10) de rayon X soit fixe dans l'espace, c'est-à-dire conserve sa position dans l'espace,
dans lequel la première commande (18) d'entraînement commande l'entraînement motorisé du système (4) de levage automatiquement en conservant l'axe (14) central fixe dans l'espace et/ou
dans lequel la deuxième commande (19) d'entraînement commande l'entraînement motorisé des roues (2) automatiquement en conservant l'axe (14) central fixe dans l'espace.

3. Procédé suivant la revendication 2, dans lequel le déplacement de compensation de l'arceau en C (6) s'effectue exclusivement sous la forme d'un déplacement vertical et/ou horizontal de l'arceau en C (6).

4. Procédé suivant la revendication 2 ou 3, dans lequel, pour effectuer le déplacement de compensation de l'arceau en C (6), on commande exclusivement des entraînements motorisés du système (4) de levage et des roues (2).

5. Procédé suivant l'une des revendications 2 à 4, dans lequel le déplacement de compensation de l'arceau en C (6) s'effectue exclusivement pendant un déplacement du récepteur de rayon X, le rapprochant de l'objet (11) ou l'en éloignant.

6. Programme d'ordinateur, qui effectue un procédé suivant l'une des revendications 2 à 5, lorsqu'il est réalisé sur un dispositif (20) d'ordinateur, qui fait partie d'une unité centrale de commande d'un dispositif (1) radiographique mobile.
